# EUROPEAN PATENT SPECIFICATION

(11) **EP 2 328 534 B1**
(45) Date of publication and mention of the grant of the patent: **06.11.2013**
(21) Application number: 09741509.5
(22) Date of filing: 21.09.2009
(51) Int. Cl.: A61F 13/20, A61F 13/34, A61F 15/00

(54) **WRAPPER HAVING A CORD RESERVOIR**
VERPACKUNG MIT KABELRESERVOIR
EMBALLAGE COMPORTANT UN RÉSERVOIR DE CORDON

(30) Priority: 25.09.2008 US 237758
(43) Date of publication of application: 08.06.2011
(73) Proprietor: The Procter & Gamble Company, Cincinnati, OH 45202 (US)
(72) Inventor: WASSON, Matthew, Howard, Cincinnati Ohio 45212 (US); WENDT, Holger, H-1054 Budapest (HU); KARAPASHA, Nancy, Cleves Ohio 45002 (US)
(74) Representative: Briatore, Andrea
(86) International application number: PCT/US2009/057607
(87) International publication number: WO 2010/036584

(56) References cited:
- EP-A2- 0 988 847
- WO-A2-2009/034556
- US-A1- 2003 065 300
- US-A1- 2003 073 970

## Description

### FIELD OF THE INVENTION

The invention relates to wrappers for feminine devices, and more particularly to wrappers for feminine devices having a cord reservoir.

### BACKGROUND OF THE INVENTION

Feminine devices, such as tampons and pessaries, are generally used by women within the vagina for feminine needs, such as, for example, to absorb menstrual or other body exudates, for pelvic support, and/or for other feminine needs. Such feminine products can be inserted into the vagina by using an applicator, or can be inserted digitally using a finger. Typically, these types of feminine products can be removed after use when the user pulls a withdrawal member attached to the tampon.

Feminine devices can be individually packaged. Individual packaging can improve hygiene, for example, by keeping the devices from being soiled by dust, unintended touching, and the like. The individual packaging can include a wrapper, such as a wrapper made of plastic film. Digital tampons, for example, can be tightly packaged in wrappers that can assist in sustaining the shape of the enclosed tampon over time under possible changes of temperature and/or humidity, in addition to facilitating ease of carrying the tampon before use.

A user may prefer that this withdrawal cord remain easily accessible during use, such that the user can easily grasp the string when she is ready to remove the tampon. In the case of applicator tampons, the withdrawal cord can be extended through the plunger, for example. Digital tampons, on the other hand, typically require that the withdrawal member be compressed against and/or into the tampon body so that the tampon and cord can be tightly packed within the wrapper. When the user opens and removes the wrapper to prepare the tampon for insertion, the withdrawal cord can remain compacted and/or can be tangled. The user may have to unpack the withdrawal cord and search for the end of it in order to extend the cord prior to insertion.

As such, it would be desirable to provide a wrapper such as, e.g., a wrapper for a feminine device, with improved withdrawal cord management.

Documents EP 988 847 and WO 2009/034 556 describe tampons having systems for cord management, but still the need remains for further improved systems.

### SUMMARY OF THE INVENTION

Wrappers having cord reservoirs are provided. A feminine device having an insertion end, a withdrawal end, and a withdrawal member, the withdrawal member having a proximal end nearest the withdrawal end of the feminine device and a distal end disposed opposite the proximal end is provided. The feminine device is at least partially enclosed by a wrapper. The wrapper has a first end disposed adjacent to the insertion end, a second end opposite the insertion end, and a longitudinal axis. The wrapper has a cord reservoir at the second end of the wrapper, the cord reservoir being adapted to contain at least 50% of the withdrawal member within the cord reservoir. In addition, the cord reservoir has a length measured along the longitudinal axis from the distal end of the feminine device to the second end of the wrapper of at least 1 mm. The first end of the wrapper tightly conforms to the outer surface of the tampon. In certain embodiments, the withdrawal member can have a withdrawal element releasably joined to the distal end.

A method for making a wrapped feminine device is also provided.

### BRIEF DESCRIPTION OF THE DRAWINGS

Figure 1 is a plan view of a wrapped tampon.
Figure 2 is a plan view of a wrapped tampon.
Figure 3 is a plan view of a wrapped tampon.
Figure 4 is a plan view of a wrapped tampon.

### DETAILED DESCRIPTION OF THE INVENTION

The present invention relates to a wrapper having a cord reservoir. The wrapper can at least partially enclose a feminine device, such as, e.g., a tampon or pessary. In certain embodiments, the feminine device can have a withdrawal member and the wrapper can include a cord reservoir that can enclose at least part of a withdrawal member. In addition, in certain embodiments, the wrapper can include a cord reservoir that is adapted to enclose the withdrawal member.

Preparing a feminine device for insertion can represent a major part of a user's experience with the device. Therefore, if the feminine device is difficult to prepare for insertion, such as, e.g., enclosed in a wrapper that can be difficult to remove, or with a tightly packed withdrawal cord, a user's experience can be adversely affected. For example, digital tampons can include a withdrawal member that can be compacted, e.g., wound, looped, folded, entangled, or the like, when the tampon is contained in the wrapper. The withdrawal member can need to be deployed, e.g., stretched, straightened, loosened, unraveled, disentangled, or the like, by a user after wrapper removal and prior to insertion of the device. Grasping the withdrawal member can be awkward and can disrupt hygienic device handling due to the user touching the device with their fingers while the user tries to prepare the device. As such, wrappers of the present invention can provide an improved experience to a user, such as, e.g., by improving deployment of the withdrawal member and/or by improving the user's wrapper removal experience.

As used herein, the term "feminine device" includes devices useful for feminine needs, such as, e.g., incontinence articles and absorbent articles useful for feminine needs, such as articles that typically can be intended for feminine use internally, such as, e.g., within a user's vagina. Internal feminine devices can include, for example, tampons, pessaries, and contraceptive devices.

As used herein, the term "tampon" refers to any type of absorbent structure that can be inserted into the vaginal canal or other body cavity, such as, e.g., for the absorption of fluid, to aid in wound healing, and/or for the delivery of materials, such as moisture or active materials such as medicaments.

As used herein, the term "vaginal canal" refers to the internal genitalia of the human female in the pudendal region of the body. The terms "vaginal canal" or "within the vagina" as used herein are intended to refer to the space located between the introitus of the vagina (sometimes referred to as the sphincter of the vagina) and the cervix.

As used herein, "applicator" refers to a device or implement that facilitates the insertion of a feminine device, such as, e.g., a tampon or pessary, into an external orifice of a mammal, such as, e.g., the vaginal canal. Exemplary applicators include telescoping, tube and plunger, and compact applicators.

As used herein, the term "digital tampon" refers to a tampon that is intended to be inserted into a vagina with a user's finger and generally without aid of an applicator.

As used herein, the term "pessary" refers to any type of structure for the purpose of reducing urine leakage and/or supporting a prolapsed uterus and/or bladder. Such pessaries can have any variety of shapes and sizes including cylinder, ovate, spherical, tubular, annual rings, "U" shaped, cup shaped, rings, cubes or donut shaped, and can function in any suitable manner, such as, e.g., by direct application of support, lever force, expansion of the device by selection of material, and/or by inflation of the device.

As used herein, the term "wrapper" refers to a structure that can be formed of one or more wrapper materials and can at least partially enclose one or more devices, such as, e.g., a tampon and/or pessary, for packaging purposes. It can be constituted of one connected piece of one or more wrapper materials or it can be made from multiple pieces of one or more wrapper materials that can be joined together.

As used herein, the term "fixedly joined" refers to a connection that is not meant to be easily released, such as, e.g., a connection formed using a substantially permanent seal and/or a connection that cannot be unattached without at least partially destroying one of the attached components.

As used herein, the term "releasably joined" refers to a connection that is meant to be easily released.

As used herein, the term "associated" refers to two elements that are near but not connected, such as, for example, two elements that may be touching but not joined.

As used herein, the term "insertion end" refers to the portion of the feminine device including the end that is intended to enter the vaginal canal first when inserting the feminine device into the vaginal canal.

As used herein, the term "withdrawal end" refers to the portion of the feminine device opposite the insertion end. In certain embodiments, the withdrawal end can include the end is intended to exit the vaginal canal first when the feminine device is removed from the vagina.

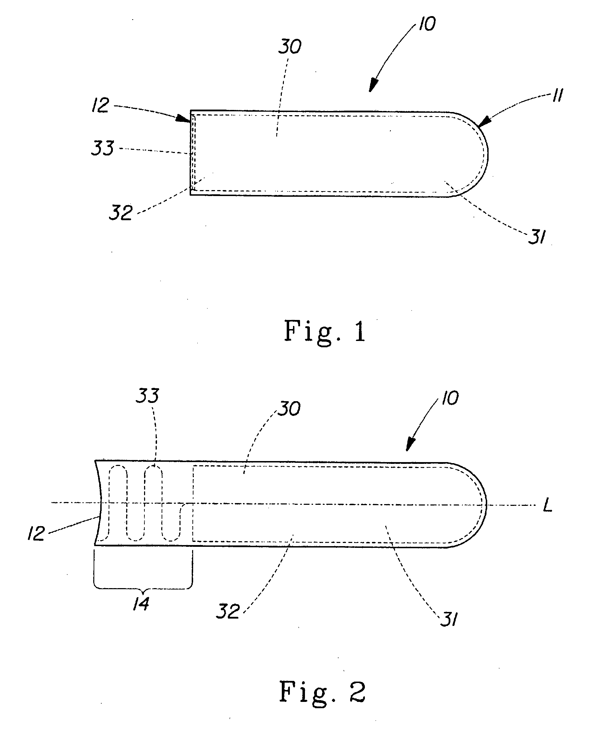

Figure 1 shows one embodiment of a prior art wrapper 10. The wrapper 10 has a first end 11 and a second end 12. As shown in Figure 1, the wrapper 10 can at least partially enclose a tampon 30. The tampon 30 can have an insertion end 31, a withdrawal end 32, and a withdrawal member 33. The withdrawal member 33 is tightly packed against the body of the tampon 30.

Figure 2 shows one embodiment of a wrapper 10. The wrapper 10 has a first end 11 and a second end 12. As shown in Figure 2, the wrapper 10 can at least partially enclose a tampon 30. The tampon 30 can have an insertion end 31, a withdrawal end 32, and a withdrawal member 33. The wrapper 10 has a cord reservoir 14. As shown in Figure 2, the withdrawal member 33 is loosely packed within cord reservoir 14.

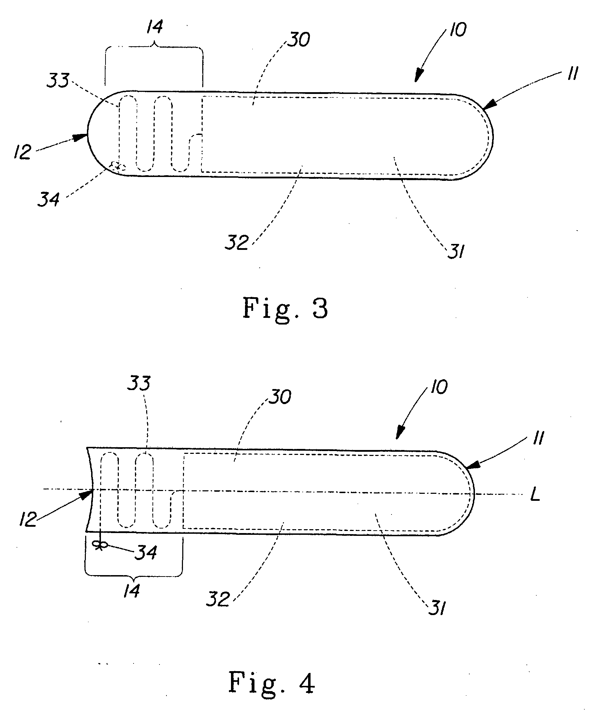

Figures 3 and 4 show embodiments of a wrapper 10. The wrapper 10 has a first end 11 and a second end 12. The wrapper 10 can at least partially enclose a tampon 30. The tampon 30 can have an insertion end 31, a withdrawal end 32, and a withdrawal member 33. The wrapper 10 has a cord reservoir 14. As shown in Figures 3 and 4, the withdrawal member 33 can be loosely packed within cord reservoir 14. In addition, in certain embodiments, the withdrawal member 33 can include a releasably joined withdrawal element 34, such as, for example, a bead, a bow, or other suitable element. The withdrawal element 34 can be pulled to extend withdrawal member 33, after which withdrawal element 34 will disconnect from withdrawal member 33. In certain embodiments, withdrawal element 34 can then be discarded along with wrapper 10. In addition, as shown in Figure 3, withdrawal element 34 can be packaged along with withdrawal member 33 within cord reservoir 14. Alternatively, as shown in Figure 4, a portion of withdrawal member 33 can protrude through wrapper 10 allowing withdrawal element 34 to be positioned outside the wrapper 10, such as, e.g., on the exterior of wrapper 10. In certain embodiments, the withdrawal element 34 can also function as an opening device.

The cord reservoir can have any suitable size provided it is adapted to contain at least 50% of the withdrawal member. The cord reservoir is provided at the withdrawal end of the tampon and can include a length measured along the longitudinal axis of the wrapper. The length can he any suitable length, such as, for example, greater than about 1 mm, such as, e.g., from about 1 mm to about 10 mm, from about 2 mm to about 8 nun, from about 3 mm to about 6 mm, from about 4 mm to about 5 mm, or any other suitable length. In addition, the cord reservoir can have any suitable width measured perpendicular to the length. For example, in certain embodiments, the cord reservoir can have a width approximately equal to the width of the tampon, or any other suitable width.

In certain embodiments, at least about 60%, at least about 70%, at least about 80%, at least about 90%, and/or substantially the entire withdrawal member can be disposed within the cord reservoir. In certain embodiments, the distal end of the withdrawal member can be disposed within the cord reservoir.

The wrapper can include any suitable opening device. Suitable opening devices include, e.g., one or more perforations, such as, e.g., disclosed in U.S. Patent No. 6,955,665, one or more depressions, such as, e.g., disclosed in European Patent No. EP 597446, a tear tape, a line of sealing, such as, e.g., disclosed in U.S. Patent No. 4,648,513, and the like. In certain embodiments, the one or more opening devices can be provided around at least a portion of a perimeter or a length of the wrapped feminine device.

The wrapper material used can be any material suitable for use for wrapping a feminine device. Suitable wrapper materials include, e.g., plastic films made of cellophane, polyethylene, polypropylene, polyester, polystyrene, PET (polyethylenetherephthalate), polyamide, polyvinylchloride, ethylene-vinyl acetate copolymer and the like; synthetic or natural elastomers, e.g., rubber; generally occlusive materials such as metallic foils, e.g., aluminum foil; non-occlusive or porous materials, such as nonwoven materials, woven materials, scrims, meshes, and papers; or any other suitable materials. In certain embodiments, the wrapper can include one or more flexible polymeric films, such as, for example, films having a thickness of less than about 1 mm.

The wrapper can be made using any suitable technique, including, for example, heat-shrinking, heat sealing, adhesives, pressure, stretching, lamination, coating, gluing, embossing, crimping, sewing, stitching, entangling, mechanical interlocking, cold pressure welding, ultrasonic bonding, and/or combinations thereof.

The wrapper can include one or more tabs. Suitable tabs include, e.g., tabs set forth in, e.g., U.S. Patent Nos. 4,648,513; 5,133,457; and 6,955,665. A tab of a wrapper of the present invention can have any suitable shape, such as e.g., a flap, a strip, a cord, a twist, a flare, a frill, a fringe, a ribbon, a loop, or the like. It can be constructed as part of the wrapper material and/or by attaching an additional material to the wrapper. It can be formed using any suitable technique, including, for example, pressing, heat sealing, adhesives, gluing, lamination, embossing, crimping, sewing, stitching, entangling, raveling, twisting, folding, mechanical interlocking, welding, ultrasonic bonding, fusing, and/or combinations thereof.

The feminine device can have a withdrawal member. The withdrawal member can be any suitable configuration, such as, e.g., one or more cords, strings, finger covers, ribbons, an extension of a material of the device, or combinations thereof. The withdrawal member can be made of any suitable material, such as, e.g., cotton and rayon. The withdrawal member can optionally be provided with a secondary absorbent member. Suitable secondary absorbent members are described in, e.g., U.S. Patent No. 6,258,075.

In certain embodiments, the withdrawal member can include a withdrawal element. The withdrawal element can be provided in any suitable location, such as, e.g., at the distal end of the withdrawal member. In certain embodiments, the withdrawal element can be releasably attached to the withdrawal member such that the withdrawal element can be disconnected from the withdrawal member prior to insertion of the feminine device by the user. The withdrawal element can be any suitable element, such as, e.g., a bead, a sparkle, a bow, a gem, or any other suitable element. In certain embodiments, the withdrawal element can also function as an opening device.

In certain embodiments, the feminine device can be a tampon. The tampon can include a pledget that can include a single material or a combination of materials. The materials for the tampon can be formed into a fabric, web, or batt that is suitable for use in the tampon by any suitable process such as, for example, airlaying, carding, wetlaying, hydroentangling, or other known techniques.

The pledget can be constructed from a wide variety of liquid-absorbing materials commonly used in absorbent articles. Such materials include, for example, rayon (such as GALAXY rayon (a tri-lobed rayon) or DANUFIL rayon (a round rayon), both available from Kelheim Fibres GmbH of Kelheim, Germany), cotton, folded tissues, woven materials, nonwoven materials, synthetic and/or natural fibers or sheeting, comminuted wood pulp, which is generally referred to as airfelt, foams, or combinations of these materials. Examples of other suitable materials include: creped cellulose wadding; meltblown polymers including coform; chemically stiffened, modified or cross-linked cellulosic fibers; synthetic fibers such as crimped polyester fibers; peat moss; foam; tissue including tissue wraps and tissue laminates; or any equivalent material or combinations of materials, or mixtures of these. Additionally, superabsorbent materials, such as superabsorbent polymers or absorbent gelling materials can be incorporated into the tampon.

The pledget can have any suitable shape, size, material, or construction prior to compression and/or shaping. For example, the pledget can include a rolled, tubed, or flat construction of an absorbent that can be a circle, an oval, a semi-circle, a triangle, a chevron shape, an H shape, a bow-tie shape, or any other suitable shape, such as, e.g., shapes described in, for example, U.S. Patent Nos. 3,738,364; 5,911,712; 6,740,070; 6,887,266; and 6,953,456.

In certain embodiments, all or a portion of the tampon can be compressed into a substantially cylindrical configuration, however, other shapes are possible. These can include shapes having a cross section or cross-section element that can be described as rectangular, triangular, trapezoidal, semi-circular, hourglass, or other suitable shapes. In certain embodiments, the tampon can have a radially compressed rolled construction. The tampon can be constructed by rolling and radially compressing a pledget. In addition, or alternatively, the tampon can include an asymmetric insertion end, such as, e.g., tampons disclosed in U.S. Pat. Appln. Nos. 11/526,041 and 11/525,513.

In certain embodiments, the tampon can have a length extending between the withdrawal end and the insertion end. In such an embodiment, the tampon may have a patterned impression in the withdrawal end, the insertion end, or both. In addition, the body portion of the tampon may have substantially no patterned impression. According to certain embodiments, a tampon with a patterned end may have a body portion with creases formed during compression of the uncompressed pledget, but such creases may not be considered a patterned impression. In addition, the patterned impression can be one or more colors and/or can include one or more design elements and the one or more design elements can be the same or different colors.

A tampon can comprise one or more overwraps. The overwrap can be any suitable material, such as, for example, rayon, cotton, bicomponent fibers, polyethylene, polypropylene, other suitable natural or synthetic fibers known in the art, and mixtures thereof. In certain embodiments, the tampon can comprise an overwrap material that substantially encloses the compressed tampon. In certain embodiments, the overwrap can extend from the withdrawal end of the tampon.

In certain embodiments, the tampon can include a finger pocket and/or a finger indent at the withdrawal end of the tampon. Suitable finger pockets arc described in, for example, U.S. Patent Application US 2010-0056981 A1, or U.S. Patent No. 6,283,952. In addition, or alternatively, the tampon can include an overwrap that extends from the withdrawal end and forms a finger cover. In certain embodiments, the tampon can include an overwrap that extends from the withdrawal end and forms an absorbent skirt. In addition, the excess of the overwrap can be at least partially disposed in a finger pocket or a finger indent and can be pulled out to provide a finger cover or an absorbent skirt when the user prepares the tampon for insertion. In certain embodiments, the excess of the overwrap can be joined with the withdrawal cord and disposed in the finger pocket or the finger indent along with the withdrawal cord, such as, for example, in U.S. Patent Appln. US 2008-0077107 and US 2008-0077108.

The cord reservoir can be constructed in any suitable manner. For example, in certain embodiments, in the case of a tampon, the withdrawal member can then be bundled about the base of the tampon during the tampon formation process. In certain embodiments, a finger pocket can be formed by applying a compression member either directly to the base of the tampon or over all or a portion of the bundled withdrawal member. The tampon can then be loaded into a wrapper tube that can be sealed at each end. In certain embodiments, the tampon can be held or otherwise manipulated such that a cord reservoir is provided at the withdrawal end of the wrapper. In addition, the wrapper can be sealed at the withdrawal end to form a cord reservoir having any suitable shape, such as, e.g., a flat shape, a concave shape, a convex shape, a rounded shape, a squared off shape, a pointed shape, or any other suitable shape.

The present invention is further illustrated by the following example, which should not be construed as limiting in any way.

### EXAMPLES

### EXAMPLE 1

This example demonstrates the creation of a loose cord in a preformed cord reservoir using vibration.

### Materials and Methods

Pledgets including a fibrous mat constructed of 100% rayon fibers (Galaxy trilobal rayon) and a spun-bond polypropylene overwrap were prepared. The pledgets included a withdrawal cord made of 100% cotton.

The pledgets were radially compressed with the cord bundled up at the base end of the tampon and were axially compressed using a header and a pushrod. The pushrod was applied over the bundled withdrawal cord to form a finger pocket.

Forty-one wrappers were made. The wrappers were sealed in a tube configuration and on the base end. Tampon pledgets were loaded into the wrappers base first. The body of the tampon was held in place to create an approximately 5 mm reservoir at the base end of the wrapper and the wrapper tip was sealed.

The wrapped tampons were then vibrated to loosen the withdrawal cord using either a vibration table or vibratory bowls.

Ten products were vibrated on a vibration table. The frequency was adjusted in 5 Hz increments and the products were vibrated for 30 seconds at a time. Frequencies that excited the cord to move were recorded. At the frequency that excited the cord the most, the frequency was adjusts +4 Hz in 1 Hz increments. Products were then assessed for cord looseness.

Thirty-one additional products were vibrated in vibratory bowls. The frequency was adjusted in 5 Hz increments and the products were vibrated for 30 seconds at a time. Frequencies that excited the cord to move were recorded. At the frequency that excited the cord the most, the frequency was adjusts +4 Hz in 1 Hz increments. Products were then assessed for cord looseness.

Cord looseness was evaluated by measuring and recording cord distance from the base of the tampon after vibration. The wrapper was opened and the cord hanging from the base after wrapper removal was measured. The cord bundle length was measured using the furthest cord strand from the tampon base.

Results are shown in Table 1.

**Table 1: Cord measurements (mm)**

| **All products:** | | | | | | |
|---|---|---|---|---|---|---|
| | | Before vibration | | After vibration | | |
| tampon # | Cord Reservoir Length | Cord from wrapper base | Cord bundle length | Cord from wrapper base | Cord from tampon | Cord bundle delta |
| 1 | 6 | 5 | 1 | 5 | 1 | 0 |
| 2 | 6 | 2 | 4 | 0 | 6 | 2 |
| 3 | 5 | 0 | 5 | 0 | 5 | 0 |
| 4 | 4 | 1 | 3 | 1 | 3 | 0 |
| 5 | 3 | 0 | 3 | 0 | 3 | 0 |
| 6 | 4 | 1 | 3 | 0 | 4 | 1 |
| 7 | 3 | 0 | 3 | 0 | 3 | 0 |
| 8 | 4 | 0 | 4 | 0 | 4 | 0 |
| 9 | 3 | 0 | 3 | 0 | 3 | 0 |
| 10 | 5 | 0 | 5 | 0 | 5 | 0 |
| 11 | 5 | 0 | 5 | 0 | 5 | 0 |
| 12 | 6 | 0 | 6 | 0 | 6 | 0 |
| 13 | 3 | 1 | 2 | 0 | 3 | 1 |
| 14 | 7 | 1 | 6 | 0 | 7 | 1 |
| 15 | 5 | 0 | 5 | 0 | 5 | 0 |
| 16 | 4 | 0 | 4 | 0 | 4 | 0 |
| 17 | 5 | 1 | 4 | 0 | 5 | 1 |
| 18 | 4 | 1 | 3 | 0 | 4 | 1 |
| 19 | 6 | 1 | 5 | 0 | 6 | 1 |
| 20 | 4 | 2 | 2 | 0 | 4 | 2 |
| 21 | 5 | 1 | 4 | 0 | 5 | 1 |
| 22 | 6 | 1 | 5 | 1 | 5 | 0 |
| 23 | 5 | 0 | 5 | 0 | 5 | 0 |
| 24 | 5 | 0 | 5 | 0 | 5 | 0 |
| 25 | 4 | 4 | 0 | 1 | 3 | 3 |
| 26 | 5 | 1 | 4 | 0 | 5 | 1 |
| 27 | 5 | 0 | 5 | 0 | 5 | 0 |
| 28 | 6 | 0 | 6 | 0 | 6 | 0 |
| 29 | 5 | 0 | 5 | 0 | 5 | 0 |
| 30 | 4 | 2 | 2 | 1 | 3 | 1 |
| 31 | 5 | 0 | 5 | 0 | 5 | 0 |
| average st dev | 4.74 | 0.81 | 3.94 | 0.29 | 4.45 | 0.52 |
| | 1.03 | 1.19 | 1.48 | 0.94 | 1.26 | 0.77 |

As shown in Table 1, a cord reservoir averaging 4.74 mm in length was successfully created by changing the loading position of the tampon into the wrapper. Table 1 also shows that pre-vibration, the average cord bundle distance from the tampon is 3.9 mm.

Table 2 shows the ability of the vibration to further loosen the cord from the base.

**Table 2: Cord measurements (mm)**

| **Product where the cord was not at the base pre vibration testing:** | | | | | | |
|---|---|---|---|---|---|---|
| | | Before vibration | | After vibration | | |
| tampon # | Cord Reservoir Length | Cord from wrapper | Cord bundle length | Cord from wrapper | Cord from tampon | Cord bundle delta |
| 4 | 4 | 1 | 3 | 1 | 3 | 0 |
| 6 | 4 | 1 | 3 | 0 | 4 | 1 |
| 13 | 3 | 1 | 2 | 0 | 3 | 1 |
| 14 | 7 | 1 | 6 | 0 | 7 | 1 |
| 17 | 5 | 1 | 4 | 0 | 5 | 1 |
| 18 | 4 | 1 | 3 | 0 | 4 | 1 |
| 19 | 6 | 1 | 5 | 0 | 6 | 1 |
| 21 | 5 | 1 | 4 | 0 | 5 | 1 |
| 22 | 6 | 1 | 5 | 1 | 5 | 0 |
| 26 | 5 | 1 | 4 | 0 | 5 | 1 |
| 2 | 6 | 2 | 4 | 0 | 6 | 2 |
| 20 | 4 | 2 | 2 | 0 | 4 | 2 |
| 30 | 4 | 2 | 2 | 1 | 3 | 1 |
| 25 | 4 | 4 | 0 | 1 | 3 | 3 |
| 1 | 6 | 5 | 1 | 5 | 1 | 0 |
| average st dev | 4.87 | 1.67 | 3.20 | 0.60 | 4.27 | 1.07 |
| | 1.13 | 1.23 | 1.61 | 1.30 | 1.53 | 0.80 |

The products that had cord already reaching the base of the wrapper were removed from the data set in Table 2, because once the cord is already touching the wrapper base it is not possible to increase the cord bundle length. As shown in Table 2, vibration was able to increase the cord bundle length an average of 1.1 mm in the remaining 15 products. This indicates that vibration does aid in creating a looser cord bundle for the consumer to grab.

All the data in Tables 1 and 2 are from the vibratory bowl feeder leg of the test. Both the vibratory bowl and the vibrating table legs showed similar qualitative results.

## Claims

1. A feminine device which is a digital tampon (30) having an outer surface, comprising an insertion end (31), a withdrawal end (32), and a withdrawal member (33), the withdrawal member (33) having a proximal end nearest the withdrawal end of the feminine device and a distal end disposed opposite the proximal end,
the feminine device being at least partially enclosed by a wrapper (10),
the wrapper (10) comprising a first end (11) disposed adjacent to the insertion end, a second end (12) opposite the insertion end, and a longitudinal axis,
the wrapper (10) having a cord reservoir (14) at the second end of the wrapper, the cord reservoir being adapted to contain at least 50% of the withdrawal member (33) within the cord reservoir,
wherein the cord reservoir (14) has a length measured along the longitudinal axis from the distal end of the feminine device to the second end of the wrapper of at least 1 mm,
being **characterized in that** the first end (11) of the wrapper (10) tightly conforms to the outer surface of the tampon (30).

2. The feminine device of claim 1, wherein the cord reservoir (14) has a length from 1 mm to 10 mm.

3. The feminine device of claim 1, wherein the cord reservoir (14) has a length from 2 mm to 8 mm.

4. The feminine device of claim 1, wherein the cord reservoir (14) has a length from 3 mm to 6 mm.

5. The feminine device of any preceding claims wherein the distal end of the withdrawal member (33) is contained within the cord reservoir (14).

6. The feminine device any of any preceding claims, wherein the withdrawal member (33) includes a withdrawal element (34) releasably joined to the distal end.

7. The feminine device of claim 6, wherein the withdrawal element (34) is contained within the cord reservoir.

8. The feminine device of claim 6, wherein the withdrawal element (34) is external to the cord reservoir.

9. A method for making the feminine device of any of claims 1 to 8, the method comprising:
a. providing a feminine device having an insertion end (31), a withdrawal end (32), and a withdrawal member (33), the withdrawal member having a proximal end nearest the withdrawal end of the feminine device and a distal end disposed opposite the proximal end;
b. providing a wrapper material having a first end, a second end opposite the first end, and a longitudinal axis;
c. sealing the wrapper material along the longitudinal axis to form a tube;
d. sealing the wrapper material at the second end (12) to form a wrapper;
e. inserting the withdrawal end (32) of the feminine device into the wrapper (10), leaving more than 1 mm of space at the second end of the wrapper to form a cord reservoir (14),
f. sealing the wrapper (10) at the first end to form a wrapped feminine device.

## Patentansprüche

1. Damenhygienevorrichtung, die aus einem digitalen Tampon (30) mit einer Außenoberfläche besteht und ein Einführende (31), ein Entnahmeende (32) und ein Entnahmeelement (33) umfasst, wobei das Entnahmeelement (33) ein proximales Ende aufweist, das sich am nächsten zum Entnahmeende der Damenhygienevorrichtung befindet, und ferner ein distales Ende aufweist, das dem proximalen Ende gegenüberliegt,
wobei die Damenhygienevorrichtung zumindest teilweise von einer Schutzhülle (10) umschlossen ist,
wobei die Schutzhülle (10) ein erstes Ende (11) aufweist, das angrenzend an das Einführende angeordnet ist, ein zweites Ende (12), das dem Einführende gegenüberliegt, und eine Längsachse,
wobei die Schutzhülle (10) ein Bändchenreservoir (14) am zweiten Ende der Schutzhülle aufweist, wobei das Bändchenreservoir so konzipiert ist, dass sich mindestens 50 % des Entnahmeelements (33) innerhalb des Bändchenreservoirs befinden,
wobei das Bändchenreservoir (14) eine entlang der Längsachse gemessene Länge vom distalen Ende der Damenhygienevorrichtung bis zum zweiten Ende der Schutzhülle von mindestens 1 mm aufweist.
**dadurch gekennzeichnet, dass** sich das erste Ende (11) der Schutzhülle (10) straff an die Außenoberfläche des Tampons (30) fügt.

2. Damenhygienevorrichtung nach Anspruch 1, wobei das Bändchenreservoir (14) eine Länge von 1 mm bis 10 mm aufweist.

3. Damenhygienevorrichtung nach Anspruch 1, wobei das Bändchenreservoir (14) eine Länge von 2 mm bis 8 mm aufweist.

4. Damenhygienevorrichtung nach Anspruch 1, wobei das Bändchenreservoir (14) eine Länge von 3 mm bis 6 mm aufweist.

5. Damenhygienevorrichtung nach einem der vorgenannten Ansprüche, wobei sich das distale Ende des Entnahmeelements (33) innerhalb des Bändchenreservoirs (14) befindet.

6. Damenhygienevorrichtung nach einem der vorgenannten Ansprüche, wobei das Entnahmeelement (33) ein Entnahmeelement (34) beinhaltet, das freigebbar mit dem distalen Ende verbunden ist.

7. Damenhygienevorrichtung nach Anspruch 6, wobei sich das Entnahmeelement (34) innerhalb des Bändchenreservoirs befindet.

8. Damenhygienevorrichtung nach Anspruch 6, wobei sich das Entnahmeelement (34) außerhalb des Bändchenreservoirs befindet.

9. Verfahren zur Herstellung der Damenhygienevorrichtung nach einem der Ansprüche 1 bis 8, wobei das Verfahren Folgendes umfasst:
a) das Bereitstellen einer Damenhygienevorrichtung mit einem Einführende (31), einem Entnahmeende (32) und einem Entnahmeelement (33), wobei das Entnahmeelement ein proximales Ende aufweist, das dem Entnahmeende der Damenhygienevorrichtung am nächsten gelegen ist, und ein distales Ende, das dem proximalen Ende gegenüberliegt;
b) Bereitstellen eines Schutzhüllenmaterials mit einem ersten Ende, einem dem ersten Ende gegenüberliegenden zweiten Ende, und einer Längsachse;
c) Verschweißen des Schutzhüllenmaterials entlang der Längsachse zum Bilden einer Röhre;
d) Verschweißen des Schutzhüllenmaterials am zweiten Ende (12) zum Bilden einer Schutzhülle;
e) Einführen des Entnahmeendes (32) der Damenhygienevorrichtung in die Schutzhülle (10), wobei am zweiten Ende der Schutzhülle mehr als 1 mm Platz gelassen wird, um ein Bändchenreservoir (14) zu bilden,
f) Verschweißen der Schutzhülle (10) an dem ersten Ende, um eine umhüllte Damenhygienevorrichtung zu bilden.

## Revendications

1. Dispositif féminin qui est un tampon digital (30) ayant une surface extérieure, comprenant une extrémité d'insertion (31), une extrémité de retrait (32) et un membre de retrait (33), ledit membre de retrait (33) ayant une extrémité proximale le plus près de l'extrémité de retrait du dispositif féminin et une extrémité distale disposée à l'opposé de l'extrémité proximale,
le dispositif féminin étant au moins partiellement recouvert par une enveloppe (10),
l'enveloppe (10) comprenant une première extrémité (11) disposée adjacente à l'extrémité d'insertion, une seconde extrémité (12) opposée à l'extrémité d'insertion et un axe longitudinal,
l'enveloppe (10) ayant un réservoir pour cordon (14) au niveau de la seconde extrémité de l'enveloppe, le réservoir pour cordon étant conçu pour contenir au moins 50 % du membre de retrait (33) à l'intérieur du réservoir pour cordon,
ledit réservoir pour cordon (14) ayant une longueur mesurée le long de l'axe longitudinal à depuis l'extrémité distale du dispositif féminin jusqu'à la seconde extrémité de l'enveloppe d'au moins 1 mm.
étant **caractérisé en ce que** la première extrémité (11) de l'enveloppe (10) épouse étroitement la surface extérieure du tampon (30).

2. Dispositif féminin selon la revendication 1, dans lequel le réservoir pour cordon (14) a une longueur de 1 mm à 10 mm.

3. Dispositif féminin selon la revendication 1, dans lequel le réservoir pour cordon (14) a une longueur de 2 mm à 8 mm.

4. Dispositif féminin selon la revendication 1, dans lequel le réservoir pour cordon (14) a une longueur de 3 mm à 6 mm.

5. Dispositif féminin selon l'une quelconque des revendications précédentes dans lequel l'extrémité distale du membre de retrait (33) est contenu à l'intérieur du réservoir pour cordon (14).

6. Dispositif féminin selon l'une quelconque des revendications précédentes, dans lequel le membre de retrait (33) inclut un élément de retrait attaché de manière libérable à l'extrémité distale.

7. Dispositif féminin selon la revendication 6, dans lequel l'élément de retrait (34) est contenu à l'intérieur du réservoir pour cordon.

8. Dispositif féminin selon la revendication 6, dans lequel l'élément de retrait (34) est externe au réservoir pour cordon.

9. Procédé de fabrication du dispositif féminin selon l'une quelconque des revendications 1 à 8, ledit procédé comprenant :
a) la fourniture d'un dispositif féminin ayant une extrémité d'insertion (31), une extrémité de retrait (32) et un membre de retrait (33), le membre de retrait ayant une extrémité proximale le plus près de l'extrémité de retrait du dispositif féminin et une extrémité distale disposée à l'opposé de l'extrémité proximale ;
b) la fourniture d'un matériau d'enveloppe ayant une première extrémité, une seconde extrémité opposée à la première extrémité et un axe longitudinal ;
c) le scellement du matériau d'enveloppe le long de l'axe longitudinal pour former un tube ;
d) le scellement du matériau d'enveloppe au niveau de la seconde extrémité (12) pour former une enveloppe ;
e) l'insertion de l'extrémité de retrait (32) du dispositif féminin dans l'enveloppe (10), en laissant plus de 1 mm d'espace au niveau de la seconde extrémité de l'enveloppe pour former un réservoir pour cordon (14),
f) le scellement de l'enveloppe (10) au niveau de la première extrémité pour former un dispositif féminin enveloppé.
